(19) Europäisches Patentamt
European Patent Office
Office européen des brevets

(11) EP 1 928 833 B1

# (12) EUROPEAN PATENT SPECIFICATION

(45) Date of publication and mention of the grant of the patent:
**18.02.2009 Bulletin 2009/08**

(21) Application number: **06792301.1**

(22) Date of filing: **26.09.2006**

(51) Int Cl.:
*C07D 215/40* $^{(2006.01)}$ *C07D 401/12* $^{(2006.01)}$
*C07D 405/12* $^{(2006.01)}$ *A61K 31/4704* $^{(2006.01)}$
*A61P 25/28* $^{(2006.01)}$

(86) International application number:
**PCT/EP2006/009417**

(87) International publication number:
**WO 2007/039220 (12.04.2007 Gazette 2007/15)**

## (54) NOVEL QUINOLINE COMPOUNDS CAPABLE OF BINDING AT THE CB2 RECEPTOR

NEUE CHINOLINVERBINDUNGEN, DIE DAZU IN DER LAGE SIND, SICH AN DEN CB2-REZEPTOR ZU BINDEN

NOUVEAUX DÉRIVÉS DE QUINOLINE SUSCEPTIBLES DE SE LIER AU RÉCEPTEUR CB2

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR**

(30) Priority: **28.09.2005 GB 0519760**

(43) Date of publication of application:
**11.06.2008 Bulletin 2008/24**

(73) Proprietor: **GLAXO GROUP LIMITED**
**Greenford, Middlesex UB6 ONN (GB)**

(72) Inventors:
- **GIBLIN, Gerard, Martin, Paul**
  **Essex CM19 5AW (GB)**
- **LIVERMORE, David, George, Hubert**
  **Essex CM19 5AW (GB)**

(74) Representative: **Thornley, Rachel Mary**
**GlaxoSmithKline**
**Corporate Intellectual Property (CN9.25.1)**
**980 Great West Road**
**Brentford, Middlesex TW8 9GS (GB)**

(56) References cited:
**EP-A1- 1 142 877**
**WO-A-20/04029026**
**WO-A2-03/080580**

EP 1 928 833 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

This invention relates to novel quinoline compounds having pharmacological activity, processes for their preparation, compositions containing them and their use in the preparation of medicaments for the treatment of diseases.

Cannabinoids are a specific class of psychoactive compounds present in Indian cannabis (Cannabis sativa), including about sixty different molecules, the most representative being cannabinol, cannabidiol and several isomers of tetrahydrocannabinol. In addition to their well known psychoactive effects, over the years cannabinoids have also been used to alleviate pain.

The pathogenic mechanisms that give rise to pain symptoms can be grouped into two main categories:

- those that are components of inflammatory tissue responses (inflammatory Pain);
- those that result from a neuronal lesion of some form (Neuropathic Pain).

With the advent of molecular biological techniques, the first cannabinoid receptor was found to be located mainly in the brain, in neural cell lines, and, only to a lesser extent, at the peripheral level. In view of its location, it was called the central receptor ("CB1"). See Matsuda et al., "Structure of a Cannabinoid Receptor and Functional Expression of the Cloned cDNA," Nature, Vol. 346, pp. 561-564 (1990). The second cannabinoid receptor ("CB2") was identified in the spleen, and was assumed to modulate the non psychoactive effects of the cannabinoids. See Munro et el., "Molecular Characterization of a Peripheral Receptor for Cannabinoids," Nature, Vol. 365, pp. 61-65 (1993).

More recent data also suggests a role for CB2 receptor activation in the CNS. The CB2 receptor was thought to be restricted to the periphery, however emerging data suggests inflammatory pain-mediated induction of CB2 receptor expression in rat spinal cord which coincides with the appearance of activated microglia (Zhang et. al., 2003). Furthermore, CB2 receptor agonists have been shown to reduce mechanically evoked responses and wind-up of wide dynamic range neurones in spinal cord dorsal horn in animal models of inflammatory pain (Zhang et. al., 2003, Eur J. Neurosci. 17: 2750-2754, Nackley et. al., 2004, J. Neurophys. 92: 3562-3574, Elmes et. al., 2004, Eur. J. Neurosci. 20: 2311-2320.)

The role of CB2 in immunomodulation, inflammation, osteoporosis, cardiovascular, renal and other disease conditions is now being examined.

Based on the foregoing, there is particular interest in compounds which have activity against the CB2 receptor and such compounds are believed to offer a unique approach toward the pharmacotherapy of pain (both inflammatory and neuropathic) immune disorders, inflammation, osteoporosis, renal ischemia and other pathophysiological conditions.

In light of the fact that cannabinoids act on receptors capable of modulating different functional effects, and in view of the low homology between CB2 and CB1 receptors, a class of drugs selective for the CB2 receptor sub-type is desirable. The natural or synthetic cannabinoids currently available do not fulfil this function because they are active at both CB2 receptors.

A structurally novel class of compounds has now been found which are capable of selectively modulating the CB2 receptor. Compounds capable of selectively modulating the CB2 receptor may be antagonists, partial or full agonists, or inverse agonists.

The present invention therefore provides, in a first aspect, a compound of formula (I):

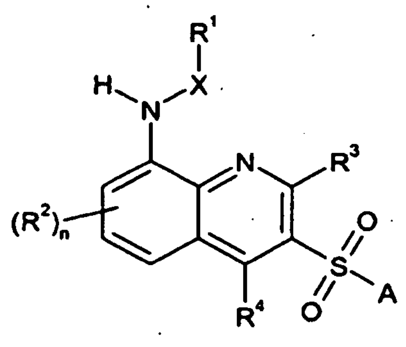

(I)

wherein:

$R^1$ represents an optionally substituted tetrahydropyranyl, morpholinyl or pyridyl;

$R^2$ represents halogen, -CN, $-CF_3$, $-OCF_3$, $-OCHF_2$, $C_{1-3}$ alkyl, $C_{1-3}$ alkoxy, $-COC_{1-3}$ alkyl, $-NR^5R^6$ or a group $-CONR^5R^6$;

$R^5$ and $R^6$ independently represent H or $C_{1-3}$ alkyl;
X represents $-(CR^7R^8)_m-$;
$R^7$ and $R^8$ at each occurrence independently represent H or $C_{1-3}$ alkyl;
m represents 1 to 4;
n represents 0 to 3;
$R^3$ and $R^4$ independently represent H, halogen, -CN, $-CF_3$, $-OCF_3$, $-OCHF_2$, $C_{1-3}$ alkyl, $C_{1-3}$ alkoxy, $-COC_{1-3}$ alkyl, $-NR^5R^6$ or a group $-CONR^5R^6$; and
A represents an optionally substituted 6 to 10 membered aryl, an optionally substituted 5 to 7 membered monocyclic heteroaryl containing 1 to 3 heteroatoms selected from O, N and S, or a 9 to 10 membered fused bicyclic heteroaryl containing 1 to 3 heteroatoms selected from O, N and S;

or a pharmaceutically acceptable salt thereof.

When $R^1$ represents an optionally substituted tetrahydropyranyl, morpholinyl or pyridyl, the tetrahydropyranyl, morpholinyl or pyridyl may be substituted by one or more (for example 1, 2 or 3) substituents, which may be the same or different, selected from the group consisting of halogen, oxygen, hydroxyl, -CN, nitro, $-NR^5R^6$, $-CONR^5R^6$, $-CF_3$, trifluoroethyl, $-OCF_3$, $-OCHF_2$, $C_{1-4}$ alkyl, $C_{1-4}$ alkoxy and $-COC_{1-4}$ alkyl.

When A is an optionally substituted 6 to 10 membered aryl, an optionally substituted 5 to 7 membered monocyclic heteroaryl, or an optionally substituted 9 to 10 membered fused bicyclic heteroaryl, it may be substituted by one or more substituents (for example 1, 2 or 3), which may be the same or different, selected from the group consisting of halogen, hydroxyl, -CN, nitro, $-NR^5R^6$, $-CONR^5R^6$, $-CF_3$, $-OCF_3$, $-OCHF_2$, $C_{1-6}$ alkyl, $C_{1-6}$ alkoxy, $-COC_{1-6}$ alkyl, $-COC_{1-6}$ alkoxy, $-NHCOC_{1-6}$ alkyl and -COOH.

As used herein, the term "alkyl" (when used as a group or as part of a group) refers to a straight or branched hydrocarbon chain containing the specified number of carbon atoms. For example, $C_{1-6}$ alkyl means a straight or branched hydrocarbon chain containing at least 1 and at most 6 carbon atoms. Examples of alkyl include, but are not limited to; methyl (Me), ethyl (Et), n-propyl, i-propyl, n-hexyl and i-hexyl.

As used herein, the term "alkoxy" (when used as a group or as part of a group) refers to an alkyl ether radical, wherein the term "alkyl" is defined above. Examples of alkoxy include, but are not limited to; methoxy, ethoxy, n-propoxy, i-propoxy, n-pentoxy and i-pentoxy.

The term 'halogen' is used herein to describe a group selected from fluorine, chlorine, bromine and iodine.

The term 'aryl' as used herein refers to a $C_{6-10}$ monocyclic or bicyclic hydrocarbon ring wherein at least one ring is aromatic. Examples of such groups include phenyl and naphthyl.

The term "heteroaryl", unless stated otherwise, is intended to mean a 5 to 7 membered monocyclic aromatic or a fused 9 to 10 membered bicyclic aromatic ring containing 1 to 3 heteroatoms selected from oxygen, nitrogen and sulfur. Suitable examples of such monocyclic aromatic rings include thienyl, furanyl, pyrrolyl, triazolyl, imidazolyl, oxazolyl, thiazolyl, oxadiazolyl, isothiazolyl, isoxazolyl, thiadiazolyl, pyrazolyl, pyrimidyl, pyridazinyl, pyrazinyl and pyridyl. Suitable examples of such fused bicyclic aromatic rings include quinolinyl, isoquinolinyl, quinazolinyl, quinoxalinyl, cinnolinyl, naphthyridinyl, indolyl, indazolyl, pyrrolopyridinyl, benzofuranyl, benzothienyl, benzimidazolyl, benzoxazolyl, benzisoxazolyl, benzothiazolyl, benzisothiazolyl, benzoxadiazolyl, benzothiadiazolyl and the like. Heteroaryl groups, as described above, may be linked to the remainder of the molecule via a carbon atom or, when present, a suitable nitrogen atom except where indicated otherwise.

It will be appreciated that wherein the above mentioned aryl or heteroaryl groups have more than one substituent, said substituents may be linked to form a ring.

In one embodiment, $R^1$ may be substituted by 1 or 2 substituents, which may be the same or different, selected from the group consisting of halogen, $-CF_3$, trifluoroethyl, $-OCF_3$, $-OCHF_2$, $C_{1-3}$ alkyl and $C_{1-3}$ alkoxy.

In one embodiment, $R^1$ represents an unsubstituted tetrahydropyranyl, (more particularly unsubstituted tetrahydro-2*H*-pyran-4-ylmethyl), an unsubstituted morpholinyl, an unsubstitued pyridyl or a methyl-substituted pyridyl.

In one embodiment, X represents $-CH_2-$ or $-C_2H_4-$.

In certain embodiments, $R^2$ represents halogen, -CN, or $C_{1-3}$ alkyl. In one embodiment, $R^2$ represents Cl or methyl.

In one embodiment, n represents 0.

In certain embodiments, $R^3$ and $R^4$ independently represent H, halogen or methyl. In one embodiment, $R^3$ and $R^4$ both represent hydrogen.

In one particular embodiment, n represents 0 and $R^3$ and $R^4$ both represent hydrogen.

In one embodiment, $R^5$ and $R^6$ independently represent hydrogen or methyl.

In certain embodiments, A represents phenyl optionally substituted by one or more (for example 1, 2 or 3) halogen atoms. In one embodiment, A represents unsubstituted phenyl.

In one embodiment there is provided a compound of formula (I) wherein:

$R^1$ represents an unsubstituted tetrahydropyranyl, an unsubstituted morpholinyl, an unsubstitued pyridyl or a methyl-substituted pyridyl;
X represents $-CH_2-$ or $-C_2H_4-$;
$R^2$ represents Cl or methyl;
n represents 0 to 3;
$R^3$ and $R^4$ independently represent H, halogen or methyl; and
A represents phenyl optionally substituted by one or more (for example 1, 2 or 3) halogen atoms;

or a pharmaceutically acceptable salt or solvate thereof.

Preferred compounds according to the invention include examples E1-E7 as shown bellow, or a pharmaceutically acceptable salt thereof.

The compounds of formula (I) can form acid addition salts thereof. It will be appreciated that for use in medicine the salts of the compounds of formula (I) should be pharmaceutically acceptable. Pharmaceutically acceptable salts include those described by Berge, Bighley and Monkhouse , J. Pharm. Sci., 1977, 66, 1-19. The term "pharmaceutically acceptable salts" includes salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc, and the like. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, trishydroxylmethyl amino methane, tripropyl amine, tromethamine, and the like. When a compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like.

Examples of pharmaceutically acceptable salts include the ammonium, calcium, magnesium, potassium, and sodium salts, and those formed from maleic, fumaric, benzoic, ascorbic, pamoic, succinic, hydrochloric, sulfuric, bismethylenesalicylic, methanesulfonic, ethanedisulfonic, propionic, tartaric, salicylic, citric, gluconic, aspartic, stearic, palmitic, itaconic, glycolic, p-aminobenzoic, glutamic, benzenesulfonic, cyclohexylsulfamic, phosphoric and nitric acids.

The compounds of formula (I) may be prepared in crystalline or non-crystalline form, and, if crystalline, may optionally be solvated, e.g. as the hydrate. This invention includes within its scope stoichiometric solvates (e.g. hydrates) as well as compounds containing variable amounts of solvent (e.g. water).

Certain compounds of formula (I) are capable of existing in stereoisomeric forms (e.g. diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The subject invention also includes isotopically-labeled compounds, which are identical to those recited in formula (I) and following, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as 3H, 11C, 14C, 18F, 1231 and 1251.

Compounds of the present invention and pharmaceutically acceptable salts of said compounds that contain the aforementioned isotopes and/or other isotopes of other atoms are within the scope of the present invention. Isotopically-labeled compounds of the present invention, for example those into which radioactive isotopes such as 3H, 14C are incorporated, are useful in drug and/or substrate tissue distribution assays. Tritiated, i.e., 3H, and carbon-14, i.e., 14C, isotopes are particularly preferred for their ease of preparation and detectability. 11C and 8F isotopes are particularly useful in PET (positron emission tomography), and 1251 isotopes are particularly useful in SPECT (single photon emission computerized tomography), all useful in brain imaging. Further, substitution with heavier isotopes such as deuterium, i.e., 2H, can afford certain therapeutic advantages resulting from greater metabolic stability, for example increased in vivo half-life or reduced dosage requirements and, hence, may be preferred in some circumstances. Isotopically labeled compounds of formula (I) and following of this invention can generally be prepared by carrying out the procedures disclosed in the Schemes and/or in the Examples below, by substituting a readily available isotopically labeled reagent for a non-isotopically labeled reagent.

Compounds of formula (I) or pharmaceutically acceptable salts thereof, may be provided by carrying out the

following process:

(a) reacting a compound of formula (II)

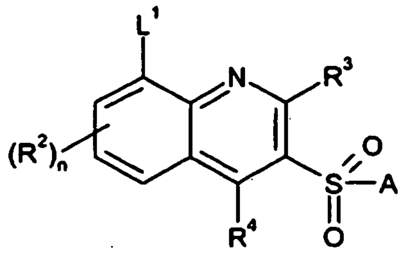

(II)

with a compound of formula (III)

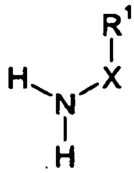

(III)

wherein $R^1$, $R^2$, $R^3$, $R^4$, n, X and A are as defined above and $L^1$ represents a suitable leaving group, such as a halogen atom (e.g. a fluorine, bromine or iodine atom) or a trifluoromethylsulfonyloxy group.

In process (a), when $L^1$ represents a fluorine atom, the process is typically carried out in the presence of a base such as potassium carbonate, and an appropriate solvent such as dimethylsulphoxide.

Process (a) may be performed in the presence of a palladium, nickel or copper catalyst, for example a mixture of a palladium source such as $Pd_2(dba)_3$ and a suitable ligand such as (R)-, (S)- or (±)-2,2'-bis(diphenylphosphino)-1,1'-binaphthyl (BINAP) or (2-dicyclohexylphosphanylphenyl)-dimethylamine or 1,1'-bis-diphenylphosphinoferrocene, together with a suitable base such as sodium *t*-butoxide, in an inert solvent such as 1,4-dioxane.

Compounds of formula (II) as defined above are described in WO 03/080580. Compounds of formula (II) wherein $L^1$ represents a fluorine atom may be provided by carrying out the following process:

(b) reacting a compound of formula (IV)

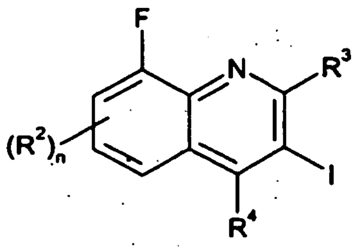

(IV)

with a compound of formula A-SO2-M, wherein $R^2$, $R^3$, $R^4$, n and A are as defined above and M is a metal residue such as sodium or potassium, in the presence of a copper (I) salt, e.g. copper (I) iodide, in a suitable solvent such as dimethylsulfoxide, optionally including a ligand such as N,N'-dimethyl-ethylene-1,2-diamine.

Compounds of formula (IV) may be provided by carrying out the following process:

(c) reacting a compound of formula (V)

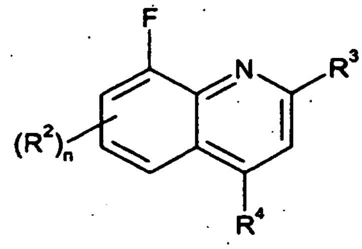

(V)

wherein $R^2$, $R^3$, $R^4$ and n are as defined above, with an iodinating agent, which can act as a source of electrophilic iodine, e.g. N-iodosuccinimide, in the presence of a solvent, e.g. acetic acid.

Compounds of formula (V) may be obtained from Orgasynth (www.orgasynth.com) or can be prepared by analogous methods.

Pharmaceutically acceptable salts may be prepared conventionally by reaction with the appropriate acid or acid derivative.

Compounds of the invention may bind to the CB2 receptor with greater affinity that to the CB1 receptor; such compounds may be particularly useful in treating CB2 receptor mediated diseases. In one embodiment compounds of formula (I) have an EC50 value at the cloned human cannabinoid CB2 receptor of at least 50 times the EC50 values at the cloned human cannabinoid CB1 receptor and/or have less than 20% efficacy at the CB1 receptor.

It is believed that compounds of the invention which bind to the CB2 receptor may be useful in the treatment of the disorders that follow. Thus, compounds of formula (I) may be useful as analgesics. For example they may be useful in the treatment of chronic inflammatory pain (e.g. pain associated with rheumatoid arthritis, osteoarthritis, rheumatoid spondylitis, gouty arthritis and juvenile arthritis) including the property of disease modification and joint structure preservation; musculoskeletal pain; lower back and neck pain; sprains and strains; neuropathic pain; sympathetically maintained pain; myositis; pain associated with cancer and fibromyalgia; pain associated with migraine; pain associated with influenza or other viral infections, such as the common cold; rheumatic fever; pain associated with functional bowel disorders such as non-ulcer dyspepsia, non-cardiac chest pain and irritable bowel syndrome (IBS); pain associated with myocardial ischemia; post operative pain; headache; toothache; and dysmenorrhea.

Compounds of the invention which bind to the CB2 receptor may also have disease modification or joint structure preservation properties in multiple sclerosis, rheumatoid arthritis, osteo-arthritis, rheumatoid spondylitis, gouty arthritis and juvenile arthritis.

Compounds of the invention which bind to the CB2 receptor may be particularly useful in the treatment of neuropathic pain. Neuropathic pain syndromes can develop following neuronal injury and the resulting pain may persist for months or years, even after the original injury has healed. Neuronal injury may occur in the peripheral nerves, dorsal roots, spinal cord or certain regions in the brain. Neuropathic pain syndromes are traditionally classified according to the disease or event that precipitated them. Neuropathic pain syndromes include: diabetic neuropathy; sciatica; non-specific lower back pain; multiple sclerosis pain; fibromyalgia; HIV-related neuropathy; post-herpetic neuralgia; trigeminal neuralgia; and pain resulting from physical trauma, amputation, cancer, toxins or chronic inflammatory conditions. These conditions are difficult to treat and although several drugs are known to have limited efficacy, complete pain control is rarely achieved. The symptoms of neuropathic pain are incredibly heterogeneous and are often described as spontaneous shooting and lancinating pain, or ongoing, burning pain. In addition, there is pain associated with normally non-painful sensations such as "pins and needles" (paraesthesias and dysesthesias), increased sensitivity to touch (hyperesthesia), painful sensation following innocuous stimulation (dynamic, static or thermal allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

Compounds of formula (I) which bind to the CB2 receptor may also be useful in the treatment of fever.

Compounds of formula (I) which bind to the CB2 receptor may also be useful in the treatment of inflammation, for example in the treatment of skin conditions (e.g. sunburn, bums, eczema, dermatitis, psoriasis); ophthalmic diseases such as glaucoma, retinitis, retinopathies, uveitis and of acute injury to the eye tissue (e.g. conjunctivitis); lung disorders (e.g. asthma, bronchitis, emphysema, allergic rhinitis, respiratory distress syndrome, pigeon fancier's disease, farmer's lung, chronic obstructive pulmonary disease, (COPD); gastrointestinal tract disorders (e.g. aphthous ulcer, Crohn's disease, atopic gastritis, gastritis varialoforme, ulcerative colitis, coeliac disease, regional ileitis, irritable bowel syndrome, inflammatory bowel disease, gastroesophageal reflux disease); organ transplantation; other conditions with an inflammatory component such as vascular disease, migraine, periarteritis nodosa, thyroiditis, aplastic anaemia, Hodgkin's

disease, sclerodoma, myaesthenia gravis, multiple sclerosis, sorcoidosis, nephrotic syndrome, Bechet's syndrome, polymyositis, gingivitis, myocardial ischemia, pyrexia, systemic lupus erythematosus, tendinitis, bursitis, and Sjogren's syndrome.

Compounds of formula (I) which bind to the CB2 receptor may also be useful in the treatment of bladder hyperrelexia following bladder inflammation.

Compounds of formula (I) which bind to the CB2 receptor may also be useful in the treatment of immunological diseases such as autoimmune diseases, immunological deficiency diseases or organ transplantation.

The compounds of formula (I) which bind to the CB2 receptor may also be effective in increasing the latency of HIV infection.

Compounds of formula (I) which bind to the CB2 receptor may also be useful in the treatment of diseases of abnormal platelet function (e.g. occlusive vascular diseases).

Compounds of formula (I) which bind to the CB2 receptor may also be useful in the treatment of neuritis, heart bum, dysphagia, pelvic hypersensitivity, urinary incontinence, cystitis or pruritis.

Compounds of formula (I) which bind to the CB2 receptor may also be useful for the preparation of a drug with diuretic action.

Compounds of formula (I) which bind to the CB2 receptor may also be useful in the treatment of impotence or erectile dysfunction.

Compounds of formula (I) which bind to the CB2 receptor may also be useful for attenuating the hemodynamic side effects of non-steroidal anti-inflammatory drugs (NSAID's) and cyclooxygenase-2 (COX-2) inhibitors.

Compounds of formula (I) which bind to the CB2 receptor may also be useful in the treatment of neurodegenerative diseases and neurodegeneration such as dementia, particularly degenerative dementia (including senile dementia, Alzheimer's disease, Pick's disease, Huntingdon's chorea, Parkinson's disease and Creutzfeldt-Jakob disease, motor neuron disease); vascular dementia (including multi-infarct dementia); as well as dementia associated with intracranial space occupying lesions; trauma; infections and related conditions (including HIV infection); dementia in Parkinson's disease ; metabolism; toxins; anoxia and vitamin deficiency; and mild cognitive impairment associated with ageing, particularly Age Associated Memory Impairment. The compounds may also be useful for the treatment of amyotrophic lateral sclerosis (ALS) and neuroinflamation.

Compounds of formula (I) which bind to the CB2 receptor may also be useful in neuroprotection and in the treatment of neurodegeneration following stroke, cardiac arrest, pulmonary bypass, traumatic brain injury, spinal cord injury or the like.

Compounds of formula (I) which bind to the CB2 receptor may also be useful in the treatment of tinnitus.

Compounds of formula (I) which bind to the CB2 receptor may also be useful in the treatment of psychiatric disease for example schizophrenia, depression (which term is used herein to include bipolar depression, unipolar depression, single or recurrent major depressive episodes with or without psychotic features, catatonic features, melancholic features, atypical features or postpartum onset, seasonal affective disorder, dysthymic disorders with early or late onset and with or without atypical features, neurotic depression and social phobia, depression accompanying dementia for example of the Alzheimer's type, schizoaffective disorder or the depressed type, and depressive disorders resulting from general medical conditions including, but not limited to, myocardial infarction, diabetes, miscarriage or abortion, etc), anxiety disorders (including generalised anxiety disorder and social anxiety disorder), panic disorder, agoraphobia, social phobia, obsessive compulsive disorder and post-traumatic stress disorder, memory disorders, including dementia, amnesic disorders and age-associated memory impairment, disorders of eating behaviours, including anorexia nervosa and bulimia nervosa, sexual dysfunction, sleep disorders (including disturbances of circadian rhythm, dyssomnia, insomnia, sleep apnea and narcolepsy), withdrawal from abuse of drugs such as of cocaine, ethanol, nicotine, benzodiazepines, alcohol, caffeine, phencyclidine (phencyclidine-like compounds), opiates (e.g. cannabis, heroin, morphine), amphetamine or amphetamine-related drugs (e.g. dextroamphetamine, methylamphetamine) or a combination thereof.

Compounds of formula (I) which bind to the CB2 receptor may also be useful in preventing or reducing dependence on, or preventing or reducing tolerance or reverse tolerance to, a dependence - inducing agent. Examples of dependence inducing agents include opioids (e.g. morphine), CNS depressants (e.g. ethanol), psychostimulants (e.g. cocaine) and nicotine.

Compounds of formula (I) which bind to the CB2 receptor may also be useful in the treatment of kidney dysfunction (nephritis, particularly mesangial proliferative glomerulonephritis, nephritic syndrome), liver dysfunction (hepatitis, cirrhosis), gastrointestinal dysfunction (diarrhoea) and colon cancer.

The term "treatment" or "treating" as used herein includes the treatment of established disorders and also includes the prophylaxis thereof. The term "prophylaxis" is used herein to mean preventing symptoms in an already afflicted subject or preventing recurrence of symptoms in an afflicted subject and is not limited to complete prevention of an affliction.

According to another aspect of the invention, we provide a compound of formula (I) which binds to the CB2 receptor, or a pharmaceutically acceptable salt thereof, for use in the treatment of a condition which is mediated by the

activity of cannabinoid 2 receptors.

According to a further aspect of the invention, we provide a preparation of a medicament for method of treating a mammal, for example a human suffering from a condition which is mediated by the activity of the CB2 receptor which comprises administering to said subject a therapeutically effective amount of a compound of formula (I) which binds to the CB2 receptor or a pharmaceutically acceptable salt thereof.

According to a further aspect of the invention we provide a preparation of a medicament for method of treating a mammal, for example a human suffering from an immune disorder, an inflammatory disorder, pain, rheumatoid arthritis, multiple sclerosis, osteoarthritis or osteoporosis which method comprises administering to said subject an effective amount of a compound of formula (I) which binds to the CB2 receptor or a pharmaceutically acceptable salt thereof.

In one embodiment the pain is selected from inflammatory pain, visceral pain, cancer pain, neuropathic pain, lower back pain, muscular skeletal, post operative pain, acute pain and migraine. For example, the inflammatory pain is pain associated with rheumatoid arthritis or osteoarthritis.

According to another aspect of the invention there is provided the use of a compound of formula (I) which binds to the CB2 receptor, or a pharmaceutically salt thereof, for the manufacture of a therapeutic agent for the treatment or prevention of a condition such as an immune disorder, an inflammatory disorder, pain, rheumatoid arthritis, multiple sclerosis, osteoarthritis or osteoporosis.

In order to use a compound of formula (I) or a pharmaceutically acceptable salt or solvate thereof for the treatment of humans and other mammals it is normally formulated in accordance with standard pharmaceutical practice as a pharmaceutical composition. Therefore in another aspect of the invention is provided a pharmaceutical composition comprising a compound of formula (I), or a pharmaceutically acceptable salt or solvate thereof, adapted for use in human or veterinary medicine.

As used herein, the expression "compounds capable of selectively modulating the CB2 receptor" means both antagonists, partial or full agonists and inverse agonists. In one embodiment the present compounds capable of selectively modulating the CB2 receptor are agonists.

In order to use the compounds of formula (I) in therapy, they will normally be formulated into a pharmaceutical composition in accordance with standard pharmaceutical practice. The present invention also provides a pharmaceutical composition, which comprises a compound of formula (I), or a pharmaceutically acceptable salt or solvate thereof, and optionally a pharmaceutically acceptable carrier.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusable solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1% to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 0.05 to 200 mg, for example 20 to 40 mg; and such unit doses will preferably be administered once a day, although administration more than once a day may be required; and such therapy may extend for a number of weeks or months.

All publications, including but not limited to patents and patent applications, cited in this specification are herein

incorporated by reference as if each individual publication were specifically and individually indicated to be incorporated by reference herein as though fully set forth.

The following Descriptions and Examples illustrate the preparation of compounds of the invention but are not intended to be limiting.

**Description 1**

**8-fluoro-3-iodoquinoline (D1)**

N-Iodosuccinimide (NIS) (229.0 g, 1.018 mol, 2.29 wt, 1.50 equivalence) was added to a stirred solution of 8-fluoroquinoline (100.0 g, 0.68 mol, 1.00 wt, 1.00 equivalence) in glacial acetic acid (AcOH) (430 ml, 4.3 vol). 8-Fluoroquinoline may be obtained from Orgasynth (www.orgasynth.com). The mixture was heated to circa 80°C under nitrogen. After 23.5 h sodium sulphite (50.0 g, 0.397 mol, 0.50 wt, 0.584 equivalence) and water (210 ml, 2.1 vol) were added and the mixture reheated to circa 80°C. After 1.5 h the mixture was allowed to cool to circa 60-65°C and seeded (100 mg, 0.1% wt). The product soon crystallised and the stirred slurry was allowed to cool over 1.5 h to ambient temperature. After 1.25 h the product was collected by vacuum filtration. The bed was washed with 1:1 acetic acid / water (2 x 300 ml, 3 vol) and water (2 x 300 ml, 2 x 3 vol). The bed was pulled dry for 5 min and the material used without further processing. A sample of the material was dried *in vacuo* at 40-45°C, to afford the desired product in 75% yield.

$^1H$ NMR, $D_4$ MeOH, 400 MHz

7.50 ppm (1H, ddd, *J* 1.5, 7.5 & 11.0 Hz), 7.58 ppm (1H, dt, *J* 5 & 8 Hz), 7.64 ppm (1H, dd, *J* 1.0 & 8.5 Hz), 8.78 ppm (1 H. t, *J* 1.5 Hz), 8.99 ppm (1H, d, *J* 2.0 Hz)

**Description 2**

**8-fluoro-3-phenylsulfonylquinoline (D2)**

A mixture of dimethylsulfoxide (500ml, 5 vol), 85% N,N'-dimethylethylenediamine (9.2 mL, 0.092 vol, 0.20 eq) and copper iodide (CuI) (7 g, 0.07 wt, 0.10 eq) was stirred at ambient temperature for 15 min to effect solution. Water (200 ml, 2 vol) was added and the mixture cooled to 22°C. Diisopropylethylamine (64mL, 0.64 vol, 1.00 eq), benzenesulfinic acid sodium salt (120.0 g, 1.20 wt, 2.00 eq) and 8-fluoro-3-iodoquinoline (D1) (123.4 g of material containing 1.4% w/w AcOH and 22% w/w $H_2O$ [equivalent to 100 g 8-fluoro-3-iodoquinoline,1.00 wt, 1.00 eq]) were added sequentially and the resulting slurry heated under nitrogen to 100°C over 1 hour, then maintained at 98-102°C for 10 hr, cooled to 22°C over 1 hour then the contents were allowed to stir for a further 1 hour. The product was collected by vacuum filtration and the cake was washed with 5:2 v/v dimethylsulfoxide - water (2 x 100ml, 2 x 2.00 vol) and water (2 x 200 ml, 2 x 2.00 vol). The bed was pulled dry and the product dried *in vacuo* at 45-50°C, to give the title compound, 78.6g, 75% yield.

$^1H$ NMR, $CDCl_3$, 400 MHz

7.54-7.67 ppm, (5H, m), 7.79 ppm (1H, d, 8.0 Hz), 8.04 ppm (2H, d, 7.5 Hz), 8.86 ppm (1H, s), 9.32 ppm (1H, d, 2.0 Hz).

**Description 3**

**3-[(4-Chlorophenyl)sulfonyl]-8-fluoroquinoline (D3)**

A mixture of 8-fluoro-3-iodoquinoline (D1) (750 mg, 2.75 mmol), sodium 4-chlorobenzenesulfinate (1.1 g, 5.5 mmol), copper (I) iodide (52 mg, 0.275 mmol) and potassium carbonate (380 mg, 2.75 mmol) was treated with *N,N'*-dimethyl-1,2-ethanediamine (49 mg, 0.55 mmol) and anhydrous dimethylsulphoxide (4 ml). The mixture was stirred at 100°C under argon for 8 hr, and cooled to 20°C. The reaction mixture was diluted with water (60 ml) and extracted with ethyl acetate (3 x 40 ml). The organic extracts were combined, washed with water (60 ml) and brine (60 ml), dried over magnesium sulphate, and evaporated to dryness. The residue was dissolved in a 1:1 mixture of dimethylsulphoxide and acetonitrile and purified by mass-directed auto-preparative chromatography using 10 minute gradients containing water and between 50% and 99% acetonitrile with 0.1 % formic acid. Product fractions were collected and evaporated to yield the title compound as a white solid (295 mg, 33%).

δH ($CDCl_3$, 400MHz) 7.51-7.69 (4H, m), 7.79 (1 H, d, J = 8 Hz), 7.96-7.99 (2H, m), 8.84 (1H, d, J = 2 Hz), 9.30 (1H, d, J = 2 Hz)

Mass spectrum: $C_{15}H_9ClFNO_2S$ requires 321; found 322 ($MH^+$)

**Description 4**

**Ethyl (2-methyl-4-pyridinyl)acetate (D4)**

To a solution containing tetrahydrofuran (THF) (100 ml) and 2M Lithium diisopropylamide in tetrahydrofuran (103 ml) was added added 2,4-Lutidine (20 g) in THF(2 0ml) at room temperature. The mixture was stirred at room temperature for 4 hours. This mixture was then transferred to a dropping funnel and added dropwise to a solution of diethylcarbonate (27 g) in tetrahydrofuran (50 ml) at room temperature and the mixture was stirred at room temperature overnight. The reaction mixture was quenched with saturated ammonium chloride solution (100 ml). The mixture was stirred for 15min and then extracted with ethyl acetate (3 x 150 ml). The organic layers were combined and dried (sodium sulphate) and evaporated. The resulting residue was chromatographed using The Flashmaster II (3 x 100 g columns) and eluting with 20% - 60% ethyl acetate / hexane. This gave a reddish brown oil (14 g). This was then taken up into concentrated hydrochloric acid (25 ml) and heated to 110°C for 1 hour. The mixture was diluted with toluene and evaporated to give a yellow oil. Ethanol (10 ml) and a drop of concentrated sulphuric acid was added and the mixture heated to 120°C for 4 hours. The mixture was evaporated, saturated brine was added and the mixture was neutralized using saturated sodium hydrogen carbonate. The resulting mixture was extracted with ethyl acetate (2 x 50 ml). The organics were combined and evaporated. The residue was purified using the Flashmaster II eluting with 40% - 70 % ethyl acetate / hexane to give the required product ethyl (2-methyl-4-pyridinyl)acetate (10.69 g)
LC/MS RT 0.89 min, m/z 180 [180+]

**Description 5**

**2-(2-methyl-4-pyridinyl)ethanol (D5)**

To a solution of ethyl (2-methyl-4-pyridinyl)acetate (D4) (0.9 g) in tetrahydrofuran (20 ml) was added lithium aluminium hydride (3.87 ml, 0.8 eq), dropwise while maintaining the internal temperature at 0°C. The mixture was allowed to stirrer at room temperature overnight. The reaction mixture was re-cooled to 0°C and quenched successively with water (5 ml), 15% sodium hydroxide solution (5 ml) and water (5 ml). The reaction mixture was then evaporated to give 2-(2-methyl-4-pyridinyl)ethanol as a pale yellow gum. (0.65 g)
NMR ( MeOD-d6) 8 2.49 (3H, s), 2.79 - 2.83 (2H, m), 3.77 - 3.81 (2H, m), 7.11 - 7.13 (1 H, m), 7.19 (1 H, s), 8.26 - 8.27 (1 H, m). Spectrum showed some minor impurities but was consistent with proposed structure
LC/MS t = 0.34 - 0.44min, [MH+] 138 consistent with the molecular formula C8H11 N O

**Description 6**

**2-[2-(2-methyl-4-pyridinyl)ethyl]-1*H*-isoindole-1,3(2*H*)-dione** (D6)

To a solution of triphenylphosphine (2 g, 1.2 eq) in tetrahydrofuran (30 ml) at 0°C was added diisopropylazodicarboxylate (1.65 ml, 1.25 eq), dropwise. The mixture was stirred at 0°C for 30 minutes. This reaction mixture was then added to a mixture containing 2-(2-methyl-4-pyridinyl)ethanol (D5) (0.9 g), phthalimide (0.93 g, 1 eq), in tetrahydrofuran (30 ml). The reaction mixture was stirred at room temperature overnight. The mixture was concentrated, and the residue was taken up into ethyl acetate (100 ml) and was washed with saturated bicarbonate (100 ml) and brine (100 ml). The organic layer was concentrated and chromotographed on the Flashmaster II eluting with 50%- 100% ethyl acetate / hexane. The product contained triphenylphosphine. The product was taken up into 1 M HCl (50 ml), and extracted with ethyl acetate (2 x 50 ml). The acidic layer was then basified using solid sodium hydrogen carbonate. This was then extracted with ethyl acetate to give 2-[2-(2-methyl-4-pyridinyl)ethyl]-1H-isoindole-1,3(2H)-dione (0.5 g).
NMR (MeOD-d6) δ 2.44 (3H, s), 2.89 - 3.02( 2H, m), 3.93 - 3.96 (2H, m), 7.09 - 7.10 (1H, m), 7.18 (1H, s), 7.77 - 7.83 (4H, m), 8.23 - 8.24 (1 H, d). Spectrum showed some minor impurities but was consistent with proposed structure
LC/MS t = 1.41min, [MH+] 267 consistent with the molecular formula C16H14N2O2

**Description 7**

**[2-(2-methyl-4-pyridinyl)ethyl]amine (D7)**

To a solution of 2-[2-(2-methyl-4-pyridinyl)ethyl]-1H-isoindole-1,3(2H)-dione (D6) (0.5 g) in ethanol (30 ml) was added hydrazine hydrate 55% in water (5 ml). The mixture was then refluxed for 4 hours. The reaction mixture was then evaporated and the residue taken in ethyl acetate (50 ml) and washed with 1 M hydrochloric acid solution (3 x 25 ml). The acid layer was re-extracted with ethyl acetate (50 ml). The acid layer was then basified using solid sodium hydrogen

carbonate. The mixture was evaporated and the solid obtained was stirred in methanol (2 x 50 ml), the solid was filtered off and the methanol was evaporated to give the required product [2-(2-methyl-4-pyridinyl)ethyl]amine (0.287 g)
NMR (MeOD-d6) δ 2.50-2.55 (>3H, m), 2.74 - 2.80 (>2H, m), 2.89 - 2.91 (1H, m), 7.10 - 7.13 (1H, m), 7.18 - 7.20 (1H, m), 8.25 - 8.29 (1H, m). Showed some minor impurities but was consistent with proposed structure.
LC/MS t = 0.32min, [MH+] 138 consistent with the molecular formula C8H12N2

**Example 1**

**3-(Phenylsulfonyl)-*N*-(tetrahydro-2*H*-pyran-4-ylmethyl)-8-quinolinamine (E1)**

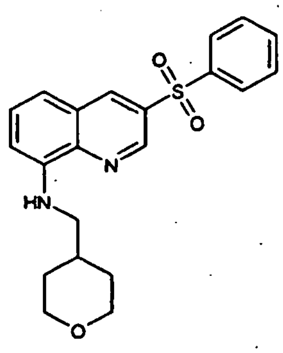

A mixture of 8-fluoro-3-(phenylsulfonyl)quinoline (D2) (58 mg, 0.2 mmol), potassium carbonate (120 mg, 0.8 mmol) and (tetrahydro-2*H*-pyran-4-ylmethyl)amine hydrochloride (Carbogen, free base also available from Combi-Blocks) (60 mg, 0.4 mmol) in dimethylsulphoxide (1 ml) was heated in a microwave vial at 150C for 6 hr. The mixture was cooled, diluted with water (75 ml) and aqueous sodium bicarbonate (25 ml) and extracted with diethyl ether (4 x 50 ml). The organic extracts were combined, washed with aqueous sodium bicarbonate (50 ml) and brine (50 ml), dried by filtration through a hydrophobic frit and evaporated to yield a gum. This material was dissolved in a mixture of dimethylsulphoxide (0.90 ml) and acetonitrile (0.90 ml) and purified by mass-directed auto-preparative chromatography using 10 minute gradients containing water and between 50% and 99% acetonitrile with 0.1 % formic acid. Product fractions were collected and evaporated to yield the title compound as a yellow solid (24 mg, 31%).
δH ($CDCl_3$, 400MHz) 1.43 (2H, dq, J = 4Hz, 12Hz), 1.76 (2H, dd, J = 2Hz, 12Hz), 1.95-2.02 (1H, m,), 3.22 (2H, t, J = 6Hz), 3.40 (2H, dt, J = 2Hz, 12Hz), 4.00 (2H, dd, J = 4Hz, 12Hz), 6.25 (1H, br t, J = 6 Hz), 6.78 (1H, d, J = 8Hz), 7.13 (1H, dd, J = 1Hz, J = 8Hz), 7.47-7.61 (4H, m), 8.02 (2H, m), 8.68 (1H, d, J = 2Hz), 9.04 (1H, d, J = 2 Hz)
Mass spectrum: $C_{21}H_{22}N_2O_3S$ requires 382; found 383 ($MH^+$)

**Example 2**

***N*-[2-(4-Morpholinyl)ethyl]-3-(phenylsulfonyl)-8-quinolinamine hydrochloride (E2)**

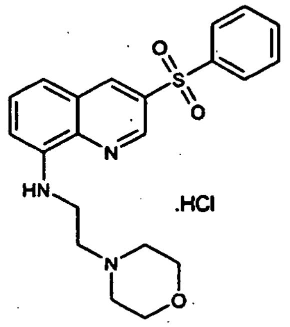

A mixture of 8-fluoro-3-(phenylsulfonyl)quinoline (D2) (56 mg, 0.2 mmol), potassium carbonate (56 mg, 0.4 mmol) and [2-(4-morpholinyl)ethyl]amine (Aldrich) (65 mg, 0.5 mmol) in dimethylsulphoxide (1 ml) was heated in a microwave vial at 150C for 4 hr. The mixture was cooled, diluted with aqueous sodium bicarbonate (75 ml) and extracted with ethyl acetate (3 x 40 ml). The organic extracts were combined, washed with aqueous sodium bicarbonate (50 ml) and brine (50 ml), dried over magnesium sulphate and evaporated to yield a gum. This material was dissolved in a mixture of dimethylsulphoxide (0.90 ml) and acetonitrile (0.90 ml) and purified by mass-directed auto-preparative chromatography using 10 minute gradients containing water and between 50% and 99% acetonitrile with 0.1% formic acid. Product fractions were collected and evaporated to yield a gum which was dissolved in ether (5 ml) and treated with 1 M hydrogen chloride in ether. The mixture was evaporated to yield the title compound as an orange solid (45 mg, 57%).
δH ($CD_3OD$, 400MHz) 3.26-3.33 (2H, m), 3.46-3.61 (4H, m), 3.74-3.83 (4H, m), 4.04-4.07 (2H, m), 7.04 (1H, d, J = 8Hz), 7.37 (1H, d, J = 8Hz), 7.56-7.70 (4H, m), 8.06-8.09 (2H, m), 8.86 (1 H, d, J = 2Hz), 9.11 (1H, d, J = 2Hz)
Mass spectrum: $C_{21}H_{23}N_3O_3S$ requires 397; found 398 ($MH^+$)

**Example 3**

**3-[(4-Chlorophenyl)sulfonyl]-*N*-(tetrahydro-2*H*-pyran-4-ylmethyl)-8-quinolinamine (E3)**

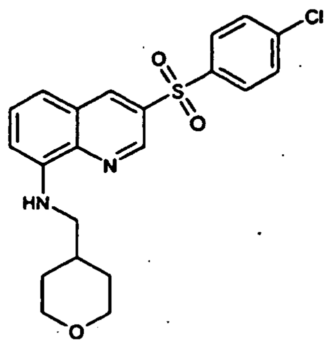

A mixture of 3-[(4-chlorophenyl)sulfonyl]-8-fluoroquinoline (D3) (64 mg, 0.2 mmol), potassium carbonate (212 mg, 1.6 mmol) and (tetrahydro-2*H*-pyran-4-ylmethyl)amine hydrochloride (Carbogen, free base also available from Combi-Blocks) (60 mg, 0.4 mmol) in dimethylsulphoxide (1 ml) was heated in a microwave vial at 150C for 4 hr. The mixture was cooled, diluted with aqueous sodium bicarbonate (75 ml) and extracted with ethyl acetate (3 x 40 ml). The organic extracts were combined, washed with aqueous sodium bicarbonate (60 ml) and brine (60 ml), dried over magnesium sulphate and evaporated to yield a gum containing a mixture of the title compound and N-(tetrahydro-2*H*-pyran-4-ylmethyl)-3-({4-[(tetrahydro-2*H*-pyran-4-ylmethyl)amino]phenyl}sulfonyl)-8-quinolinamine. This material was dissolved in a mixture of dimethylsulphoxide (0.90ml) and acetonitrile (0.90 ml) and purified by mass-directed auto-preparative chromatography using 10 minute gradients containing water and between 50% and 99% acetonitrile with 0.1 % formic acid. Product fractions were collected and evaporated to yield the title compound as a yellow solid (17 mg, 20%).
δH ($CDCl_3$, 400MHz) 1.44 (2H, dq, J = 4Hz, 12Hz), 1.76 (2H, dd, J = 2Hz, 12 Hz), 1.95-2.02 (1 H, m), 3.22 (2H, t, J = 6Hz), 3.40 (2H, dt, J = 2Hz, 12Hz), 4.01 (2H, dd, J = 4Hz, 12Hz), 6.25 (1H, br t, J = 6Hz), 6.79 (1H, d, J = 7Hz), 7.13 (1H, dd, J = 8Hz), 7.48-7.52 (3H, m), 7.94 (2H, m), 8.66 (1H, d, J = 2Hz), 9.01 (1H, d, J = 2Hz)
Mass spectrum: $C_{21}H_{21}ClN_2O_3S$ requires 416/418; found 417/419 ($MH^+$)

**Example 4**

**3-(Phenylsulfonyl)-*N*-(3-pyridinylmethyl)-8-quinolinamine** (E4)

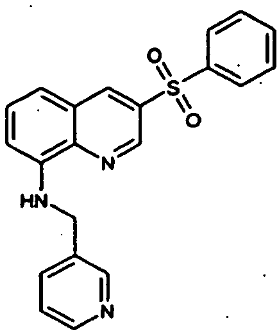

A mixture of 8-fluoro-3-(phenylsulfonyl)quinoline (D2) (56 mg, 0.2 mmol), potassium carbonate (56 mg, 0.4 mmol) and (3-pyridinylmethyl)amine (Aldrich) (50 mg, 0.46 mmol) in dimethylsulphoxide (1 ml) was heated in a microwave vial at 150C for 6 hr. The mixture was cooled, diluted with aqueous sodium bicarbonate (50 ml) and extracted with ethyl acetate (3 x 25 ml). The organic extracts were combined, washed with aqueous sodium bicarbonate (30 ml) and brine (30 ml), dried over magnesium sulphate and evaporated to yield a gum. This material was dissolved in a mixture of dimethylsulphoxide (0.90 ml) and acetonitrile (0.90 ml) and purified by mass-directed auto-preparative chromatography using 10 minute gradients containing water and between 30% and 85% acetonitrile with 0.1% formic acid. Product fractions were collected and evaporated to yield an orange solid (12 mg, 18%).
δH ($CD_3OD$, 400MHz) 4.66 (2H, s), 6.77 (1H, d, J = 8Hz), 7.25 (1H, d, J = 8Hz), 736-7.45 (2H, m), 7.56-7.69 (3H, m), 7.86 (1H, d, J = 8Hz), 8.07 (2H, dd, J = 2Hz, 7Hz), 8.27 (1H, s), 8.40 (1H, d, J = 4Hz), 8.57 (1H, s), 8.81 (1H, d, J = 2Hz), 9.00 (1H, d, J = 2Hz)
Mass spectrum: $C_{21}H_{17}N_3O_2S$ requires 375; found 376 ($MH^+$)

**Example 5**

**3-(Phenylsulfonyl)-*N*-[2-(4-pyridinyl)ethyl]-8-quinolinamine hydrochloride (E5)**

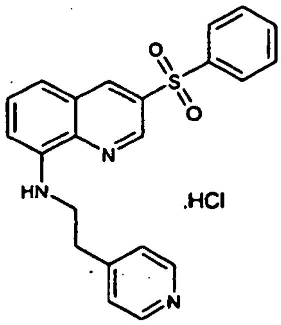

A mixture of 8-fluoro-3-(phenylsulfonyl)quinoline (D2) (56 mg, 0.2 mmol), potassium carbonate (56 mg, 0.4 mmol) and [2-(4-pyridinyl)ethyl]amine (TCI-JP, also available from Fluorochem) (50 mg, 0.4 mmol) in dimethylsulphoxide (1 ml) was heated in a microwave vial at 150C for 6 hr. The mixture was cooled, diluted with aqueous sodium bicarbonate (50 ml) and extracted with ethyl acetate (3 x 25 ml). The organic extracts were combined, washed with aqueous sodium bicarbonate (30 ml) and brine (30 ml), dried over magnesium sulphate and evaporated to yield a gum. This material was dissolved in a mixture of dimethylsulphoxide (0.90 ml) and acetonitrile (0.90 ml) and purified by mass-directed auto-preparative chromatography using 10 minute gradients containing water and between 30% and 85% acetonitrile with, 0.1 % formic acid. Product fractions were collected and evaporated to yield a gum. This material was dissolved in ether and treated with 1 M hydrogen chloride in ether. The mixture was evaporated, dissolved in ether and re-evaporated to yield the title compound as a white solid (20 mg, 24%).
δH ($CD_3OD$, 400MHz) 3.78 (2H, t, J = 6Hz), 6.97 (1H, d, J = 8Hz), 7.26 (1H, d, J = 8Hz), 7.53 (1H, t, J = 8Hz), 7.59-7.70 (3H, m), 7.84 (2H, d, J = 6Hz), 8.05-8.07 (2H, m), 8.61 (2H, d, J = 6 Hz), 8.81 (1 H, d, J = 2Hz), 9.01 (1H, d, J = 2 Hi)

Mass spectrum: $C_{22}H_{19}N_3O_2S$ requires 389; found 390 ($MH^+$)

**Example 6**

**3-(Phenylsulfonyl)-*N*-[2-(tetrahydro-2*H*-pyran-4-yl)ethyl]-8-quinolinamine hydrochloride (E6)**

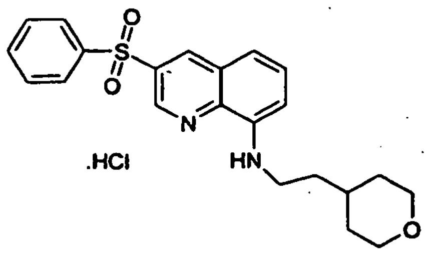

To 8-fluoro-3-(phenylsulfonyl)quinoline (D2) (300 mg), potassium carbonate (0.288 g: 2 eq) and 4-amino-ethyl [2-(tetrahydro-2*H*-pyran-4-yl)ethyl]amine (Apollo Scientific) (0.269 g, 2 eq) in DMSO(3 ml) were added to a 5 ml microwave vial. The mixture was heated at 180°C under microwave irradiation for 1 hour. The reaction mixture was diluted with sodium hydrogen carbonate (20 ml) and extracted with'ethyl acetate (3 x 20 ml). The ethyl acetate layers were combined and evaporated. The residue obtained was purified using the Flashmaster II (gradient 10-80% ethyl acetate on 50 g silica column) to give the free base of the title compound (0.255 g).
NMR (400MHz, Chloroform-d6) δ 1.31-1.41 (2H, m), 1.65-1.71 (5H, m), 3.33-3.49 (4H, m), 3.94-3.97 (2H, m), 6.93 (1H, d), 7.22 (1H, d), 7.52-7.62 (4H, m), 8.00-8.03(2H, m), 8.75 (1H, d), 9.07 (1H, d)
LC/MS, t = 3.58min, Molecular ion observed ($MH^+$) = 497 consistent with the molecular formula $C_{22}H_{24}N_2O_3S$
3-(Phenylsulfonyl)-*N*-[2-(tetrahydro-2*H*-pyran-4-yl)ethyl]-8-quinolinamine (0.255g) was taken up into methanol and treated with an excess of 2M HCl in diethyl ether to give the title compound (0.268 g)
NMR (400MHz, Chloroform-d6) 1.22-1.28 (2H, m), 1.55-1.58 (2H, m), 1.07 (1 H, bs), 1.85 (2H, bs)3.30-3.36 (2H, m), 3.42-3.52 (2H, m), 3.88-3.92 (2H, m), 6.93 (1H, d), 7.57-7.73 (4H, m), 7.85-7.87 (1H, bs), 8.04-8.10 (3H, m), 8.96 (1H, s), 9.29 (1 H, s)
LC/MS, t = 3.58min, Molecular ion observed ($MH^+$) = 497 consistent with the molecular formula $C_{22}H_{24}N_2O_3S$

**Example 7**

***N*-[2-(2-methyl-4-pyridinyl)ethyl]-3-(phenylsulfonyl)-8-quinolinamine hydrochloride (E7)**

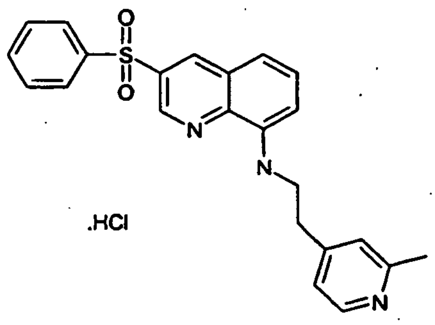

To 8-fluoro-3-(phenylsulfonyl)quinoline (200 mg) in a microwave vial was added potassium carbonate (192 mg, 2 eq), Dimethylsulfoxide (2 ml) followed by [2-(2-methyl-4-pyridinyl)ethyl]amine (D7) (190 mg, 2 eq). The mixture was sealed and heated to 180°C for 1 hour in a microwave. Saturated sodium hydrogen carbonate (2ml) was added and the mixture was extracted with ethyl acetate. The ethyl acetate layer was evaporated and the residue was purified by mass directed auto-preparative chromatography to give the free base N-[2-(2-methyl-4-pyridinyl)ethyl]-3-(phenylsulfonyl)-8-quinolinamine (26.67 mg). This was taken up into methanol and treated with 1M hydrogen chloride in ether. The

mixture was evaporated to give the required product N-[2-(2-methyl-4-pyridinyl)ethyl]-3-(phenylsulfonyl)-8-quinolinamine hydrochloride (30 mg)
NMR (DMSQ-d6) δ 2.66 (3H, s), 3.18-3.22 (2H, m), 3.67-3.70 (2H, m), 7.02-7.04 (1H, d), 7.34-7.36 (1H, d), 7.54-7.58 (1H, m), 7.63-7.86 (6H, m), 8.07-8.10 (2H, m), 8.64-8.66 (1 H, d), 8.98-8.99 (1 H, d), 9.08-9.09 (1H, d) Consistent with proposed structure.
LC/MS t = 2.09min, [MH+] 404 consistent with the molecular formula $C_{23}H_{21}N_3O_2S$

In examples where purification using mass directed auto-preparative chromatography was carried out, the following apparatus and conditions were used, unless stated otherwise:

Hardware

Waters 2525 Binary Gradient Module

Waters 515 Makeup Pump

Waters Pump Control Module

Waters 2767 Inject Collect

Waters Column Fluidics Manager

Waters 2996 Photodiode Array Detector

Waters ZQ Mass Spectrometer

Gilson 202 fraction collector

Gilson Aspec waste collector

Software

Waters MassLynx version 4 SP2

Column

Waters Atlantis, 30mm x 100mm column packed with 5(m stationary phase.

Flow rate

40mls/min.

In examples where purification is referred to as being carried out by chromatography on the Flashmaster II, the following apparatus and conditions were used:

Hardware

Biotage technologies Flashmaster II

Software

Flashmaster Version 3.1 build 2

Column

Flash Silica per packed.

Size from 2g to 100g of silica dependent on the amount of sample for purification

Flow rate

From 2ml/min to 40 ml/min dependent on the size of column used.

**Pharmacological data**

Determination of cannabinoid CB1 Receptor Agonist Activity

The cannabinoid CB1 receptor agonist activity of compounds of formula (I) was determined in accordance with the following experimental method.

Experimental Method

Yeast (Saccharomyces cerevisiae) cells expressing the human cannabinoid CB1 receptor were generated by integration of an expression cassette into the ura3 chromosomal locus of yeast strain MMY23. This cassette consisted of DNA sequence encoding the human CB1 receptor flanked by the yeast GPD promoter to the 5' end of CB1 and a yeast transcriptional terminator sequence to the 3' end of CB1. MMY23 expresses a yeast/mammalian chimeric G-protein alpha subunit in which the C-terminal 5 amino acids of Gpa1 are replaced with the C-terminal 5 amino acids of human G$\alpha$i1/23 (as described in Brown et al. (2000), Yeast 16:11-22). Cells were grown at 30°C in liquid Synthetic Complete (SC) yeast media (Guthrie and Fink (1991), Methods in Enzymology, Vol. 194) lacking uracil, tryptophan, adenine and leucine to late logarithmic phase (approximately 6 OD600/ml).

Agonists were prepared as 10 mM stocks in DMSO. EC50 values (the concentration required to produce 50% maximal response) were estimated using 4 fold dilutions dilutions of between 3- and 5-fold (BiomekFX, Beckman) into DMSO. Agonist solutions in DMSO (1% final assay volume) were transferred into black, clear bottom, microtitre plates from NUNC Greiner (96- or 384-well). Cells were suspended at a density of 0.2 OD600/ml in SC media lacking histidine, uracil, tryptophan, adenine and leucine and supplemented with 10mM 3-aminotriazole, 0.1M sodium phosphate pH 7.0, and 120$\mu$M fluorescein di-$\beta$-D-glucopyranoside (FDGlu). This mixture (50ul per well for 384-well plates, 200ul per well for 96-well plates) was added to agonist in the assay plates (Multidrop 384, Labsystems). After incubation at 30°C for 24 hours, fluorescence resulting from degradation of FDGlu to fluorescein due to exoglucanase, an endogenous yeast enzyme produced during agonist-stimulated cell growth, was determined using a Spectrofluorfluorescence microtitre plate reader ((Tecan Spectrofluor or LJL Analyst excitation wavelength: 485nm; emission wavelength: 535nm). Tecan; excitation wavelength: 485nm; emission wavelength: 535nm). Fluorescence was plotted against compound concentration and iteratively curve fitted using a four parameter fit to generate a concentration effect value:

Efficacy (Emax) was calculated from the equation

$$\text{Emax} = \text{Max[compound X]} - \text{Min[compound X]} / \text{Max[HU210]} - \text{Min[HU210]} \times 100\%$$

where Max[compound X] and Min[compound X] are the fitted maximum and minimum respectively from the concentration effect curve for compound X, and Max[HU210] and Min[HU210] are the fitted maximum and minimum respectively from the concentration effect curve for (6aR,10aR)-3-(1,1'-Dimethylheptyl)-6a,7,10,10a-tetrahydro-1-hydroxy-6,6-dimethyl-6H-dibenzo[b,d]pyran-9-methanol (HU210; available from Tocris). Equieffective molar ratio (EMR) values were calculated from the equation

$$\text{EMR} = \text{EC50 [compound X]} / \text{EC50 [HU210]}$$

Where EC50 [compound X] is the EC50 of compound X and EC50 [HU210] is the EC50 of HU210.
pEC50 is the negative log of the EC50.

Determination of cannabinoid CB2 Receptor Agonist Activity

The cannabinoid CB2 receptor agonist activity of compounds of formula (I) was determined in accordance with the following experimental method.

Experimental Method

Yeast (Saccharomyces *cerevisiae*) cells expressing the human cannabinoid CB2 receptor were generated by integration of an expression cassette into the *ura3* chromosomal locus of yeast strain MMY23. This cassette consisted of DNA sequence encoding the human CB2 receptor flanked by the yeast GPD promoter to the 5' end of CB2 and a yeast transcriptional terminator sequence to the 3' end of CB2. MMY23 expresses a yeast/mammalian chimeric G-protein alpha subunit in which the C-terminal 5 amino acids of Gpa1 are replaced with the C-terminal 5 amino acids of human Gαi1/23 (as described in Brown et al. (2000), Yeast 16:11-22). Cells were grown at 30°C in liquid Synthetic Complete (SC) yeast media (Guthrie and Fink (1991), Methods in Enzymology, Vol. 194) lacking uracil, tryptophan, adenine and leucine to late logarithmic phase (approximately 6 $OD_{600}$/ml).

Agonists were prepared as 10 mM solutions in DMSO. $EC_{50}$ values (the concentration required to produce 50% maximal response) were estimated using 4 fold dilutions of between 3- and 5-fold (BiomekFX, Beckman) into DMSO. Agonist solutions in DMSO (1% final assay volume) were transferred into black microtitre plates from NUNC Greiner (384-well). Cells were suspended at a density of 0.2 $OD_{600}$/ml in SC media lacking histidine, uracil, tryptophan, adenine and leucine and supplemented with 10mM 3-aminotriazole, 0.1 M sodium phosphate pH 7.0, and 120μM fluorescein di-β-D-glucopyranoside (FDGlu). This mixture (50ul per well) was added to agonist in the assay plates (Multidrop 384, Labsystems). After incubation at 30°C for 24 hours, fluorescence resulting from degradation of FDGlu to fluorescein due to exoglucanase, an endogenous yeast enzyme produced during agonist-stimulated cell growth, was determined using a fluorescence microtitre plate reader (Tecan Spectrofluor or LJL Analyst excitation wavelength: 485nm; emission wavelength: 535nm). Fluorescence was plotted against compound concentration and iteratively curve fitted using a four parameter fit to generate a concentration effect value.

Efficacy ($E_{max}$) was calculated from the equation

$$E_{max} = Max_{[compound\ X]} - Min_{[compound\ X]} / Max_{[HU210]} - Min_{[HU210]} \times 100\%$$

where $Max_{[compound\ X]}$ and $Min_{[compound\ X]}$ are the fitted maximum and minimum respectively from the concentration effect curve for compound X, and $Max_{[HU210]}$ and $Min_{[HU210]}$ are the fitted maximum and minimum respectively from the concentration effect curve for (6aR, 10aR)-3-(1,1'-Dimethylheptyl)-6a,7,10,10a-tetrahydro-1-hydroxy-6,6-dimethyl-6H-dibenzo[b,d]pyran-9-methanol (HU210; available from Tocris). Equieffective molar ratio (EMR) values were calculated from the equation

$$EMR = EC_{50\ [compound\ X]} / EC_{50\ [HU210]}$$

Where $EC_{50\ [compound\ X]}$ is the $EC_{50}$ of compound X and $EC_{50\ [HU210]}$ is the $EC_{50}$ of HU210.
pEC50 is the negative log of the EC50.

The compounds of Examples E1-7 were tested for cannabinoid CB2 receptor agonist activity. The compounds of Examples E1 and E3-7 had pEC50 values > 6 at the CB2 receptor. The compound of Example E2 had a pEC50 values > 5 at the CB2 receptor.

## Claims

1. A compound of formula (I):

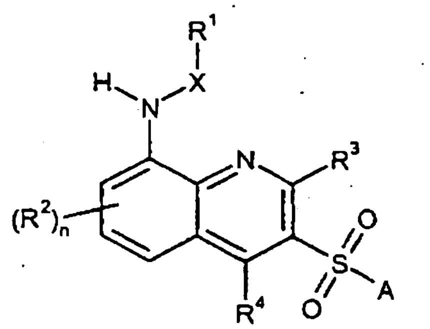

(I)

wherein:

$R^1$ represents an optionally substituted tetrahydropyranyl, morpholinyl or pyridyl;
$R^2$ represents halogen, -CN, $-CF_3$, $-OCF_3$, $-OCHF_2$, $C_{1-3}$ alkyl, $C_{1-3}$ alkoxy, $-COC_{1-3}$ alkyl, $-NR^5R^6$ or a group $-CONR^5R^6$;
$R^5$ and $R^6$ independently represent H or $C_{1-3}$ alkyl;
X represents $-(CR^7R^8)_m-$;
$R^7$ and $R^8$ at each occurrence independently represent H or $C_{1-3}$ alkyl;
m represents 1 to 4;
n represents 0 to 3;
$R^3$ and $R^4$ independently represent H, halogen, -CN, $-CF_3$, $-OCF_3$, $-OCHF_2$, $C_{1-3}$ alkyl, $C_{1-3}$ alkoxy, $-COC_{1-3}$ alkyl, $-NR^5R^6$ or a group $-CONR^5R^6$; and
A represents an optionally substituted 6 to 10 membered aryl, an optionally substituted 5 to 7 membered monocyclic heteroaryl containing 1 to 3 heteroatoms selected from O, N and S, or a 9 to 10 membered fused bicyclic heteroaryl containing 1 to 3 heteroatoms selected from O, N and S;

or a pharmaceutically acceptable salt thereof.

2. A compound of formula (I), or a pharmaceutically acceptable salt thereof, as defined in claim 1, wherein $R^1$ is substituted by 1 or 2.substituents, which may be the same or different, selected from the group consisting of halogen, $-CF_3$, trifluoroethyl, - $OCF_3$, $-OCHF_2$, $C_{1-3}$ alkyl and $C_{1-3}$ alkoxy.

3. A compound of formula (I), or a pharmaceutically acceptable salt thereof, as defined in claim 1 or 2, wherein $R^1$ represents an unsubstituted tetrahydropyranyl, an unsubstituted morpholinyl, an unsubstitued pyridyl or a methyl-substituted pyridyl.

4. A compound of formula (I), or a pharmaceutically acceptable salt thereof, as defined in any of the previous claims, wherein A represents phenyl optionally substituted by one or more halogen atoms.

5. A compound of formula (I), or a pharmaceutically acceptable salt thereof, as defined in any of the previous claims, wherein X represents $-CH_2-$ or $-C_2H_4-$.

6. A compound of formula (I), or a pharmaceutically acceptable salt thereof, as defined in any of the previous claims, wherein n represents 0 and $R^3$ and $R^4$ both represent hydrogen.

7. A compound of formula (I) as defined in claim 1 which is a compound of:

3-(Phenylsulfonyl)-*N*-(tetrahydro-2*H*-pyran-4-ylmethyl)-8-quinolinamine (E1); *N*-[2-(4-Morpholinyl)ethyl]-3-(phenylsulfonyl)-8-quinolinamine hydrochloride (E2);
3-[(4-Chlorophenyl)sulfonyl]-*N*-(tetrahydro-2*H*-pyran-4-ylmethyl)-8-quinolinamine (E3);
3-(Phenylsulfonyl)-*N*-(3-pyridinylmethyl)-8-quinolinamine (E4);
3-(Phenylsulfonyl)-*N*-[2-(4-pyridinyl)ethyl]-8-quinolinamine hydrochloride (E5);3-(Phenylsulfonyl)-*N*-[2-(tetrahydro-2*H*-pyran-4-yl)ethyl]-8-quinolinamine hydrochloride. (E6);
*N*-[2-(2-methyl-4-pyridinyl)ethyl]-3-(phenylsulfonyl)-8-quinolinamine hydrochloride (E7);

or a pharmaceutically acceptable salt thereof.

**8.** A pharmaceutical composition which comprises a compound or a pharmaceutically acceptable salt thereof as defined in any preceding claim and a pharmaceutically acceptable carrier or excipient.

**9.** A compound or pharmaceutically acceptable salt thereof as defined in any one of claims 1 to 7 for use in the treatment of: dementia; degenerative dementia, such as senile dementia, Alzheimer's disease, Pick's disease, Huntingdon's chorea, Parkinson's disease and Creutzfeldt-Jakob disease, motor neuron disease; vascular dementia such as multi-infarct dementia; dementia associated with intracranial space occupying lesions; trauma; infections and related conditions such as HIV infection; dementia in Parkinson's disease; metabolism; toxins; anoxia and vitamin deficiency; and mild cognitive impairment associated with ageing, particularly Age Associated Memory impairment.

**10.** Use of a compound or pharmaceutically acceptable salt thereof as defined in any one of claims 1 to 7 in the manufacture of a medicament for the treatment of: dementia; degenerative dementia, such as senile dementia, Alzheimer's disease; Pick's disease, Huntingdon's chorea, Parkinson's disease and Creutzfeldt-Jakob disease, motor neuron disease; vascular dementia such as multi-infarct dementia; dementia associated with intracranial space occupying lesions; trauma; infections and related conditions such as HIV infection; dementia in Parkinson's disease; metabolism; toxins; anoxia and vitamin deficiency; and mild cognitive impairment associated with ageing, particularly Age Associated Memory Impairment.

**Patentansprüche**

**1.** Verbindung der Formel (I):

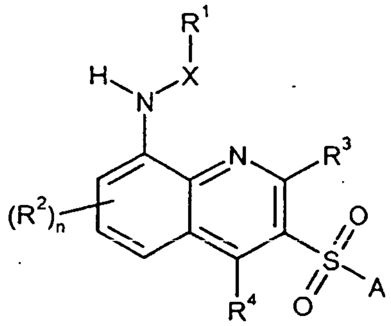

(I)

wobei
$R^1$ ein gegebenenfalls substituierter Tetrahydropyranyl-, Morpholinyl- oder Pyridylrest ist;
$R^2$ ein Halogenatom, $-CN$, $-CF_3$, $-OCF_3$, $-OCHF_2$, ein $C_{1-3}$-Alkylrest, ein $C_{1-3}$-Alkoxyrest, ein $-COC_{1-3}$-Alkylrest, $-NR^5R^6$ oder $-CONR^5R^6$ ist;
$R^5$ und $R^6$ jeweils unabhängig voneinander ein Wasserstoffatom oder ein $C_{1-3}$-Alkylrest sind;
X $-(CR^7R^8)_m-$ ist;
$R^7$ und $R^8$ bei jedem Auftreten unabhängig voneinander ein Wasserstoffatom oder ein $C_{1-3}$-Alkylrest sind;
m 1 bis 4 ist;
n 0 bis 3 ist;
$R^3$ und $R^4$ unabhängig voneinander ein Wasserstoffatom, ein Halogenatom, $-CN$, $-CF_3$, $-OCF_3$, $-OCHF_2$, ein $C_{1-3}$-Alkylrest, ein $C_{1-3}$-Alkoxyrest, ein $-COC_{1-3}$-Alkylrest, $-NR^5R^6$ oder $-CONR^5R^6$ sind; und
A ein gegebenenfalls substituierter 6- bis 10-gliedriger Arylrest, ein gegebenenfalls substituierter 5- bis 7-gliedriger monocyclischer Heteroarylrest, der 1 bis 3 Heteroatome, ausgewählt aus O, N und S, enthält, oder ein 9- bis 10-gliedriger kondensierter bicyclischer Heteroarylrest, der 1 bis 3 Heteroatome, ausgewählt aus O, N und S, enthält, ist;
oder ein pharmazeutisch verträgliches Salz davon.

**2.** Verbindung der Formel (I) oder ein pharmazeutisch verträgliches Salz davon, wie in Anspruch 1 definiert, wobei $R^1$ substituiert ist mit 1 oder 2 Substituenten, welche gleich oder verschieden sein können, ausgewählt aus Halogen,

$-CF_3$, Trifluorethyl, $-OCF_3$, $-OCHF_2$, $C_{1-3}$-Alkyl und $C_{1-3}$-Alkoxy.

**3.** Verbindung der Formel (I) oder ein pharmazeutisch verträgliches Salz davon, wie in Anspruch 1 oder 2 definiert, wobei $R^1$ ein nicht substituierter Tetrahydropyranylrest, ein nicht substituierter Morpholinylrest, ein nicht substituierter Pyridylrest oder ein Methylsubstituierter Pyridylrest ist.

**4.** Verbindung der Formel (I) oder ein pharmazeutisch verträgliches Salz davon, wie in einem der vorherigen Ansprüche definiert, wobei A ein Phenylrest ist, der gegebenenfalls mit einem oder mehr Halogenatomen substituiert ist.

**5.** Verbindung der Formel (I) oder ein pharmazeutisch verträgliches Salz davon, wie in einem der vorherigen Ansprüche definiert, wobei X $-CH_2-$ oder $-C_2H_4-$ ist.

**6.** Verbindung der Formel (I) oder ein pharmazeutisch verträgliches Salz davon, wie in einem der vorherigen Ansprüche definiert, wobei n 0 ist und $R^3$ und $R^4$ beide ein Wasserstoffatom sind.

**7.** Verbindung der Formel (I), wie in Anspruch 1 definiert:

3-(Phenylsulfonyl)-N-(tetrahydro-2*H*-pyran-4-ylmethyl)-8-chinolinamin (E1); N-[2-(4-Morpholinyl)ethyl]-3-(phenylsulfonyl)-8-chinolinaminhydrochlorid (E2); 3-[(4-Chlorphenyl)sulfonyl]-*N*-(tetrahydro-2*H*-pyran-4-ylmethyl)-8-chinolinamin (E3); 3-(Phenylsulfonyl)-*N*-(3-pyridinylmethyl)-8-chinolinamin (E4); 3-(Phenylsulfonyl)-*N*-[2-(4-pyridinyl)ethyl]-8-chinolinaminhydrochlorid (E5); 3-(Phenylsulfonyl)-*N*-[2-(tetrahydro-2*H*-pyran-4-yl)ethyl]-8-chinolinaminhydrochlorid (E6); *N*-[2-(2-Methyl-4-pyridinyl)ethyl]-3-(phenylsulfonyl)-8-chinolinaminhydrochlorid (E7);

oder ein pharmazeutisch verträgliches Salz davon.

**8.** Arzneimittel, welches eine Verbindung oder ein pharmazeutisch verträgliches Salz davon, wie in einem der vorherigen Ansprüche definiert, und einen pharmazeutisch verträglichen Träger oder Exzipienten umfasst.

**9.** Verbindung oder pharmazeutisch verträgliches Salz davon, wie in einem der Ansprüche 1 bis 7 definiert, zur Verwendung bei der Behandlung von: Demenz; degenerativer Demenz, wie z.B. seniler Demenz, Alzheimer-Krankheit, Pick-Krankheit, Chorea Huntington, Parkinson-Krankheit und Creutzfeld-Jakob-Krankheit, Motoneuron-Krankheit; vaskulärer Demenz, wie z.B. Multünfarkt-Demenz; Demenz in Verbindung mit intrakranieller Raumforderung; Trauma; Infektionen und verwandter Zustände, wie z.B. HIV-Infektion; Demenz bei Parkinson-Syndrom; Stoffwechsel; Giften; Anoxie und Vitaminmangel; und leichter kognitiver Beeinträchtigung verbunden mit dem Altern, insbesondere altersverbundener Gedächtnisbeeinträchtigung.

**10.** Verwendung einer Verbindung oder eines pharmazeutisch verträglichen Salzes davon, wie in einem der Ansprüche 1 bis 7 definiert, zur Herstellung eines Medikaments zur Behandlung von: Demenz, degenerativer Demenz, wie z.B. seniler Demenz, Alzheimer-Krankheit, Pick-Krankheit, Chorea Huntington, Parkinson-Krankheit und Creutzfeld-Jakob-Krankheit, Motoneuron-Krankheit; vaskulärer Demenz, wie z.B. Multiinfarkt-Demenz; Demenz in Verbindung mit intrakranieller Raumforderung; Trauma; Infektionen und verwandter Zustände, wie z.B. HIV-Infektion; Demenz bei Parkinson-Syndrom; Stoffwechsel; Giften; Anoxie und Vitaminmangel; und leichter kognitiver Beeinträchtigung verbunden mit dem Altern, insbesondere altersverbundener Gedächtnisbeeinträchtigung

## Revendications

**1.** Composé de formule (I) :

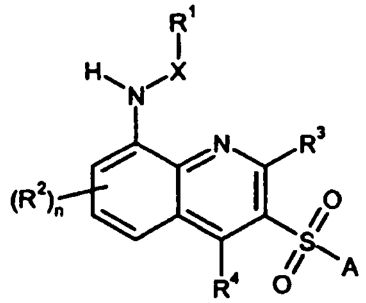

(I)

formule dans laquelle :

$R^1$ représente un groupe tétrahydropyrannyle, morpholinyle ou pyridyle, facultativement substitué ;
$R^2$ représente un atome d'halogène, un groupe -CN, $-CF_3$, $-OCF_3$, $-OCHF_2$, alkyle en $C_1$ à $C_3$, alkoxy en $C_1$ à $C_3$, -CO(alkyle en $C_1$ à $C_3$), $-NR^5R^6$ ou un groupe $-CONR^5R^6$ ;
$R^5$ et $R^6$ représentent indépendamment H ou un groupe alkyle en $C_1$ à $C_3$ ;
X représente un groupe $-(CR^7R^8)_m-$ ;
$R^7$ et $R^8$, à chaque occurrence, représentent indépendamment H ou un groupe alkyle en $C_1$ à $C_3$ ;
m a une valeur de 1 à 4 ;
n a une valeur de 0 à 3 ;
$R^3$ et $R^4$ représentent indépendamment H, un atome d'halogène, un groupe -CN, $-CF_3$, $-OCF_3$, $-OCHF_2$, alkyle en $C_1$ à $C_3$, alkoxy en $C_1$ à $C_3$, -CO(alkyle en $C_1$ à $C_3$), $-NR^5R^6$ ou un groupe $-CONR^5R^6$ ; et
A représente un groupe aryle hexa- à décagonal facultativement substitué, un groupe hétéroaryle monocyclique penta- à heptagonal facultativement substitué contenant 1 à 3 hétéroatomes choisis entre O, N et S, ou un groupe hétéroaryle bicyclique condensé nona- ou décagonal contenant 1 à 3 hétéroatomes choisis entre O, N et S ;

ou un de ses sels pharmaceutiquement acceptables.

**2.** Composé de formule (I), ou un de ses sels pharmaceutiquement acceptables, suivant la revendication 1, dans lequel $R^1$ est substitué avec 1 ou 2 substituants, qui peuvent être identiques ou différents, choisis dans le groupe consistant en des substituants halogéno, $-CF_3$, trifluoréthyle, $-OCF_3$, $-OCHF_2$, alkyle en $C_1$ à $C_3$ et alkoxy en $C_1$ à $C_3$.

**3.** Composé de formule (I), ou un de ses sels pharmaceutiquement acceptables, suivant la revendication 1 ou 2, dans lequel $R^1$ représente un groupe tétrahydropyrannyle non substitué, un groupe morpholinyle non substitué, un groupe pyridyle non substitué ou un groupe pyridyle à substituant méthyle.

**4.** Composé de formule (I), ou un de ses sels pharmaceutiquement acceptables, suivant l'une quelconque des revendications précédentes, dans lequel A représente un groupe phényle facultativement substitué avec un ou plusieurs atomes d'halogène.

**5.** Composé de formule (I), ou un de ses sels pharmaceutiquement acceptables, suivant l'une quelconque des revendications précédentes, dans lequel X représente $-CH_2-$ ou $-C_2H_4-$.

**6.** Composé de formule (I), ou un de ses sels pharmaceutiquement acceptables, suivant l'une quelconque des revendications précédentes, dans lequel n est égal à 0 et $R^3$ et $R^4$ représentent l'un et l'autre un atome d'hydrogène.

**7.** Composé de formule (I) suivant la revendication 1, qui est le composé suivant :

la 3-(phénylsulfonyl)-*N*-(tétrahydro-2*H*-pyranne-4-ylméthyl)-8-quinolinamine (E1); le chlorhydrate de *N*-[2-(4-morpholinyl)-éthyl]-3-(phénylsulfonyl)-8-quinolinamine (E2) ; la 3-[(4-chlorophényl)sulfonyl]-*N*-(tétrahydro-2*H*-pyranne-4-ylméthyl)-8-quinolinamine (E3) ; la 3-(phénylsulfonyl)-*N*-(3-pyridinylméthyl)-8-quinolinamine (E4) ; le chlorhydrate de 3-(phénylsulfonyl)-*N*-[2-(4-pyridinyl)éthyl]-8-quinolinamine (E5) ; le chlorhydrate de 3-(phénylsulfonyl)-*N*-[2-(tétrahydro-2*H*-pyranne-4-yl)éthyl]-8-quinolinamine (E6) ; le chlorhydrate de *N*-[2-(2-méthyl-4-pyridinyl)éthyl]-3-(phénylsulfonyl)-8-quinolinamine (E7) ;

ou un de ses sels pharmaceutiquement acceptables.

**8.** Composition pharmaceutique qui comprend un composé ou un de ses sels pharmaceutiquement acceptables suivant l'une quelconque des revendications, et un support ou excipient pharmaceutiquement acceptable.

**9.** Composé ou son sel pharmaceutiquement acceptable suivant l'une quelconque des revendications 1 à 7, destiné à être utilisé dans le traitement de : la démence ; une démence dégénérative telle que la démence sénile, la maladie d'Alzheimer, la maladie de Pick, la chorée de Huntington, la maladie de Parkinson et la maladie de Creutzfeldt-Jakob, une maladie neuronale motrice ; une démence vasculaire telle que la démence artériopathique ; la démence associée à des lésions occupant des espaces intracrâniens ; un traumatisme ; des infections et affections apparentées telles que l'infection par le VIH ; la démence dans la maladie de Parkinson ; un trouble du métabolisme ; des toxines ; une anoxie et une déficience vitaminique ; et l'altération cognitive bénigne associée au vieillissement, en particulier l'altération de la mémoire due à l'âge.

**10.** Utilisation d'un composé ou de son sel pharmaceutiquement acceptable suivant l'une quelconque des revendications 1 à 7, dans la production d'un médicament destiné au traitement due : la démence ; une démence dégénérative telle que la démence sénile, la maladie d'Alzheimer, la maladie de Pick, la chorée de Huntington, la maladie de Parkinson et la maladie de Creutzfeldt-Jakob, une maladie neuronale motrice ; une démence vasculaire telle que la démence artériopathique ; la démence associée à des lésions occupant des espaces intracrâniens ; un traumatisme ; des infections et affections apparentées telles que l'infection par le VIH ; la démence dans la maladie de Parkinson ; un trouble du métabolisme ; des toxines ; une anoxie et une déficience vitaminique ; et l'altération cognitive bénigne associée au vieillissement, en particulier l'altération de la mémoire due à l'âge.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03080580 A

### Non-patent literature cited in the description


- **MATSUDA et al.** Structure of a Cannabinoid Receptor and Functional Expression of the Cloned cDNA. *Nature,* 1990, vol. 346, 561-564
- **MUNRO.** Molecular Characterization of a Peripheral Receptor for Cannabinoids. *Nature,* 1993, vol. 365, 61-65
- **ZHANG.** *Eur J. Neurosci.,* 2003, vol. 17, 2750-2754
- **NACKLEY.** *J. Neurophys,* 2004, vol. 92, 3562-3574
- **ELMES.** *Eur. J. Neurosci.,* 2004, vol. 20, 2311-2320
- **BERGE ; BIGHLEY ; MONKHOUSE.** *J. Pharm. Sci.,* 1977, vol. 66, 1-19
- **BROWN et al.** *Yeast,* 2000, vol. 16, 11-22
- **GUTHRIE ; FINK.** *Methods in Enzymology,* 1991, vol. 194